# EUROPEAN PATENT APPLICATION

(11) **EP 0 629 963 A2**
(43) Date of publication of application: **21.12.1994**
(21) Application number: 94304291.1
(22) Date of filing: 14.06.1994
(51) Int. Cl.: A61B 19/00, G06F 15/42

(54) **A display system for visualization of body structures during medical procedures**

(30) Priority: 21.06.1993 US 78335
(71) Applicant: GENERAL ELECTRIC COMPANY, Schenectady, NY 12345 (US)
(72) Inventor: Dumoulin, Charles Lucian, Ballston Lake, New York 12019 (US); Darrow, Robert David, Scotia, New York 12302 (US); Adams, William John, Clifton Park, New York 12065 (US)
(74) Representative: Lupton, Frederick

(57) **Abstract**

An interactive display system superimposes radiological images on a semi-transparent screen through which a surgeon views a patient during a medical procedure. The superimposed image is derived from image data obtained with an imaging system. The radiological image is registered with the surgeon's view of the patient and displayed in real-time during a medical procedure. This allows the surgeon to view internal and external structures and the relation between them simultaneously, and adjust the procedure accordingly. A second embodiment employs stereoscopic viewing methods to provide three-dimensional representations of the radiological images superimposed on the semi-transparent screen through which the surgeon views the patient.

## Description

### BACKGROUND OF THE INVENTION

### 1. Field of the Invention

The present invention relates to a system for aiding a medical practitioner, such as a surgeon, in visualizing anatomical structures during medical procedures and more specifically to a system which allows the practitioner to view, in real time, both internal and external anatomical structures.

### 2. Discussion of Prior Art

This application is related to International application US 94/02572 "Computer Graphic and Live Video System for Enhancing Visualization of Body Structures During Surgery" and to European Patent applications 0549183 "System For Displaying Solid Cuts For Surfaces of Solid Models", 0549189 "Solid Models Generation By Span Method Using Dividing Cubes" and 0549182 "Apparatus and Method For Displaying Surgical Cuts in Three-Dimensional Models".

Presently, during surgery and other medical procedures such as endoscopy, biopsy and implantation, physicians view several static radiographic views of the patient in the operating room. Typically these are transparent film renderings of magnetic resonance (MR), computed tomography (CT), conventional X-ray images or ultrasound images. Since these images are two dimensional static images, the physicians must determine actual three-dimensional (3D) location and shape of desired internal structures within the patient from the 2D images which they are viewing. Conceptually, the surgeon constructs a 3D model of the internal structures and correlates these internal structures with visible external structures of the patient where they must cut. This is often difficult because the scale and the orientation of the 2D image may differ from what the surgeon is seeing, and the surgeon may not be able to view both the patient and the medical diagnostic images simultaneously.

Another technique employed in localization of internal structures during surgery is known as stereotactic surgery as described in "Interactive Stereotactic Surgical System for the Removal of Intracranial Tumors Utilizing the CO₂ Laser and CT-Derived Database" by B. A. Kall, P. J. Kelly, and S. J. Goerss, IEEE Transactions on Biomedical Engineering, vol. BME-32, no. 2, pp 112-116, 1985; and "Comprehensive Computer-Assisted Data Collection Treatment Planning and Interactive Surgery" by B. A. Kall, P. J Kelly, and S. J. Goerss, Medical Imaging, vol. 767 pp. 509-514, 1987. With this approach, a rigid mechanical frame is attached to the patient before a CT or MR procedure. The frame and its landmarks can be seen in the resulting images. Mechanisms on the frame position a probe at specific location within the image. The disadvantages of this approach are that the frame limits access to the patient, and the images are static images which do not follow the patient if he moves during surgery.

A third technique used for localization of internal structures is described in "A Frameless Stereotaxic Operating Microscope for Neurosurgery" by E. M. Friets, J. W. Strohbehn, J. F. Hatch, and D. W. Roberts, IEEE Transactions on Biomedical Engineering, vol. 36., no. 6, pp 608-617, June 1989.

Three dimensional models of anatomical structures can be created from data of different medical imaging modalities as described in the applications listed above. These applications describe creating and manipulating models of internal structures of patients and providing images of selected structures at desired orientations to an operator. These allow visualization of internal structures as solid models.

Currently there is a need for a system to aid physicians in surgery and other medical procedures which interactively displays computer generated representations of internal structures in correct relation with external structures of the patient.

### SUMMARY OF THE INVENTION

A feature of the present invention is to provide a system which aids in surgery by simultaneously superimposing an image of internal structures upon external structures even if the patients changes their position or if the operators change their viewing angles.

Another feature of the present invention is to provide an interactive system which displays desired internal structures upon external structures, having the same scale and viewed from the same orientation angles.

Another features of the present invention is to provide a surgeon a "heads up" display of radiological images superimposed upon a patient within his field of view.

A real-time surgery apparatus for displaying interactive internal and external images of a patient employs a semi-transparent display screen allowing an operator to simultaneously view exposed surfaces and computer generated images of a patient.

A medical imaging device obtains three-dimensional (3D) imaging data of internal structures of the patient which are fed to a workstation. The workstation creates three-dimensional (3D) computer generated models which may be manipulated without further need for the medical imaging device. Two-dimensional computer generated images of the models are interactively oriented and scaled so that their display on a semi-transparent display device coincides with the operator's visual image of the patient.

The three-dimensional position and orientation of the patient, display device and operator's eyes are monitored in real-time. This information is then used to adapt the computer generated display on the semi-transparent screen so that the visual and computer generated images consistently coincide.

### BRIEF DESCRIPTION OF THE DRAWINGS

The features of the invention believed to be novel are set forth with particularity in the appended claims. The invention itself, however, both as to organization and method of operation, together with further objects and advantages thereof, may best be understood by reference to the following description taken in conjunction with the accompanying drawing in which:

Fig. 1 is a simplified block diagram of a first embodiment of a medical display apparatus according to the present invention.

Fig. 2 is a simplified block diagram of a second embodiment of a medical display apparatus according to the present invention.

### DETAILED DESCRIPTION OF THE INVENTION

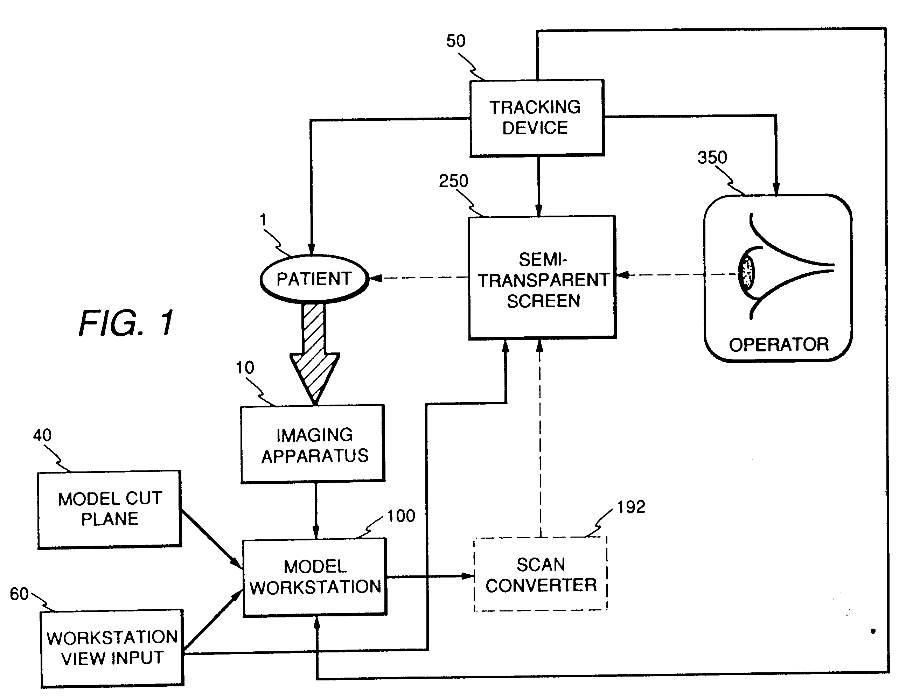

In Fig. 1, a patient 1 on which a medical procedure such as surgery is to be performed, is scanned by a medical imaging apparatus 10 which may be a magnetic resonance (MR) imaging apparatus, a computed axial tomography (CAT) apparatus, a positron emission tomography (PET) or similar imaging device capable of creating multi-dimensional volumetric data such as 3-dimensional (3-D) data, from internal structures of the patient. After imaging, apparatus 10 provides the volumetric data to a model workstation 100. Once the volumetric data has been provided to model workstation 100, further need for imaging apparatus 10 imaging apparatus is no longer required. This is important since some medical procedures need not be performed with the patient situated within the confines of an imaging apparatus, which can be constricting in the case of MR imaging. In alternative embodiments, imaging apparatus 10 may be interactively employed during the medical procedure. Model workstation 100 stores the volumetric data and creates computer generated models from the data capable of being scaled, rotated and otherwise manipulated, without the further need for imaging apparatus 10.

An operator 350, such as a physician or medical assistant, monitors patient 1. A semi-transparent screen 250 is interposed between patient 1 and operator 350. A tracking device 50 which monitors and tracks operator 350, semi-transparent screen 250 and patient 1 determines a relative roll α, pitch ϑ, and yaw φ orientation between operator 350, semi-transparent screen 250 and subject 1. Tracking device 50 may be a 6-degrees of freedom tracking device as described "The Flock of Birds" Installation and Operation Guide, Ascension Technology Corporation, July 5, 1992, in the Introduction pp. 1-3, Appendix 1, p. 89 and Appendix 3, p. 93. Tracking device 50 also determines a location (in Cartesian coordinates) of operator 350 and semi-transparent screen 250 with relation to patient 1. Patient 1 is assumed to be at the origin of the Cartesian coordinate system (x,y,z) = (0,0,0), therefore all distances relative to the patient are simply the (x,y,z) location. The location and orientation are interactively provided to model workstation 100 by tracking device 50. The location and orientation may also be provided manually to model workstation(s) in different embodiments.

Model workstation 100 processes the 3D volumetric data it receives and creates selected renderings of the data. One rendering method determines surfaces between differing types of tissue. Connectivity of similar types of tissue adjacent to one another is then determined. Differentiation of tissue types based on the nature of the signal in the three-dimensional image data is known as segmentation. When the 3-D volumetric data has been segmented into internal structures, each internal structure may be treated as a separate solid object by model workstation 100. The model workstation has the capability of selectively displaying desired internal structures, color coding structures and severing, rotating and translating internal structures in order to manipulate these images in a desired manner to provide visualization to an operator working model workstation 100.

An alternative rendering method generates two-dimensional projections of selected features within the three-dimensional data set is described in European Patent Application 0506302 "Projection Methods for Producing Two-Dimensional Images from Three-Dimensional Data". For example, two-dimensional projection angiograms can be extracted from a three-dimensional phase contrast or time-of-flight magnetic resonance angiogram. Several projection algorithms are possible. These include the detection of the maximum pixel intensity along a selected projection ray through the three-dimensional data, determination of the average pixel intensity of a selected projection ray and the determination of the standard deviation of all pixels along a selected projection ray.

Model workstation 100 receives input data from a model cut plane input device 40 and a workstation view input device 60 to select the method of displaying internal structures of patient 1. Model cut plane input device 40 and a workstation view input device 60 may be a computer pointing device such as a mouse or trackball, or any input device which indicates planes in which to cut the images and a viewing angle and scale. Tracking device 50 provides relative orientation data between operator 350 and patient 1 and scaling, which allows model workstation 100 to synthesize an interactive computer generated image of internal structures of patient 1 and have it coincide with the real-time scene viewed by operator 350. This interactive computer generated image may be converted from a computer monitor signal, such as an RGB computer monitor signal, to a video format by passing it through a scan converter 192 (shown in phantom), depending on the display format of semi-transparent screen 250. The computer generated image is provided to semi-transparent screen 250 which is interposed between patient 1 and operator 350. Semi-transparent screen 250 provides a desired mixture of external structures of patient 1 seen by operator 350 and the computer generated image from model workstation 100. Semi-transparent screen 250 may receive input signals from the operator, for example, through workstation view input 60. This may involve the degree of transparency of each image, or any of various other special effects. One very useful special effect is a movable window which has 0% transparency (100% opaqueness) superimposed upon another image. When the window image is of internal structures superimposed upon external structures, it creates the illusion of external structures cut away within the window, exposing underlying internal structures. Other conventional video special effects may also be employed. Semi-transparent screen 250 allows operator 350 to visualize both internal and external structures simultaneously. The resulting scene witnessed by operator 350 is an interactive real-time image of patient 1, even if the patient moves during the medical procedure. Since internal structures and their relation to exposed external structures are simultaneously displayed, a surgeon using the present invention will perceive a very accurate indication of where he should cut through external structures to arrive at a desired internal structure while avoiding vital internal structures.

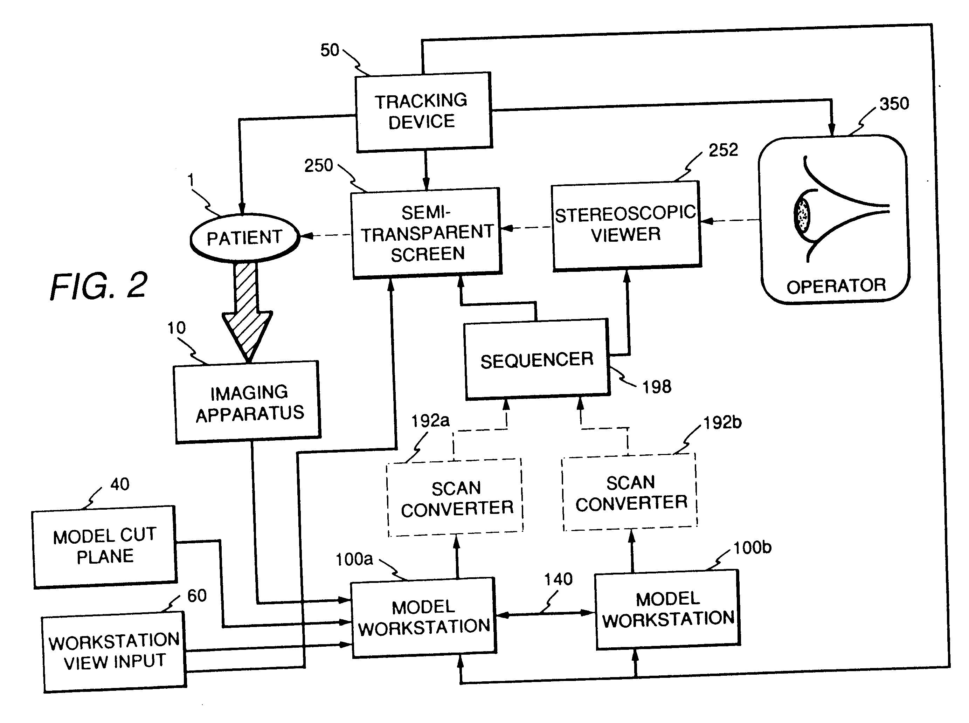

Figure 2 illustrates an alternative embodiment of the present invention in which operator 350 receives a stereoscopic view of internal and external structures of patient 1. Each of the eyes of operator 350 differs in its orientation with relation to the patient therefore a different view is provided to each eye. Tracking device 50 tracks the relative location (x₁,y₁,z₁) and orientation angle (α₁, φ₁, ϑ₁) between a first eye of operator 350 and patient 1. Tracking device 50 also tracks a second location (x₂,y₂,z₂) and orientation (α₂,φ₂,ϑ₂) between a second eye of operator 350 and patient 1. The second location and orientation can be measured independently by tracking device 50; or the location and orientation of the first eye can be used to compute the location and orientation of the second eye. The locations and orientations are fed to a first model workstation 100a, and a second model workstation 100b which create a right and left computer graphic image at locations (x₁,y₁,z₁), (x₂,y₂,z₂), respectively, and orientations (α₁, φ₁, ϑ₁) (α₂,φ₂,ϑ₂), respectively, corresponding to the views of each eye of operator 350, respectively. Control signals from model cut plane 40, workstation view input 60 and imaging apparatus 10 can be sent to both first model workstation 100a, which in turn propagates the control signals to second model workstation 100b via communications path 140, or alternatively, the control signals can be sent directly to both model workstations directly.

The left and right computer generated image, pertaining to a left and right view, respectively, are converted to video format if required by semi-transparent screen 250. This is accomplished by scan converters 192a, 192b (shown in phantom) which pass the converted computer generated signals to a sequencer 198. Sequencer 198 may be a conventional video sequencer as described in "Portable, Low Cost Devices for Videotaping, Editing and Displaying Field Sequential Stereoscopic Motion Pictures and Video" by M. Starks, Stereoscopic Displays and Applications Proc. SPIE Vol. 1256, pp. 266-271, 1990.

Sequencer 198 passes the left computer generated image to semi-transparent screen 250. Sequencer 198 then passes the right computer generated image to semi-transparent screen 250. Sequencer 198 alternates many times per second, in synchronization, between right and left views.

The image displayed on semi-transparent screen 250 is time multiplexed to produce an image to the left eye and right eye of the operator in an alternating fashion. A stereoscopic viewer 252 is synchronized to a sequencer 198 and operates to block the vision of the operator's left or right eye allowing the opposite eye to view the image on semi-transparent screen 250 for an instant and vice-versa. This allows operator 350 to see the left image with the left eye while the right eye sees nothing and the right image with the right eye while the left eye sees nothing in rapid succession. This creates a stereoscopic illusion, adding the dimension of depth perception in viewing the image displayed on semi-transparent screen 250. Depth perception is very valuable in surgery since it adds a dimension that assists in visually localizing structures, which is especially important in complex, delicate surgery.

For both embodiments of the invention the computer image must be registered (coincide) with the external structures as viewed by operator 350. Initialization may be accomplished by manual input from the operator to rotate, translate and scale the computer generated image(s) until they coincide with the scene observed through the semi-transparent screen, or by employing tracking device 50 to set initial parameters.

Once the 3D model and the visual image of patient 1 are aligned, tracking device 50 keeps the view angles and-field of view consistent. This allows real-time interactive synchronization between the operator's view of patient 1 and the computer generated image(s).

Since the image of each internal structure can be segmented into what resembles a solid object, it may be manipulated as a solid object. In the case of structures of a patient, a surgeon may acquire data from the patient by medical imaging, then plan surgery by manipulating the models to plan a desired result before surgery. This is common in complex reconstructive surgery. Once the plan is determined, it may be stored and played back during surgery. The images of internal structures are interactively oriented and scaled to coincide with the actual patient.

A user employs as a model cut plane input device, a workstation view input device (40, 60 of Figs. 1 and 2, respectively) to select planes in which to cut the structures in the model, to select a three-dimensional orientation of the model, and to select screen cut planes which define a workstation viewing region. The model workstation can incorporate a clipping circuit to determine points within the model cut planes, a rotation circuit to rotate points and normal vectors, a segmentation processor, and a shading circuit which determines shading based upon the orientation of the normal vector at each point. In addition a screen clipping circuit can be used to determine points within a region defined by the screen cut planes. The model workstation also can include a display circuit to create video signals which, when propagated to a suitable display device, generate images of multiple surfaces that are within the desired display region and the screen cut planes.

In radical surgery such as ablative surgery, or massive trauma cases, there is little structure which remains to correctly determine what a normal anatomy should be. In these cases, an additional model workstation may have a model of normal structures stored which may be mixed with the other images being displayed to act as a guide in reconstructive surgery. This may be implemented by additional workstations or model manipulation boards.

In the present embodiments of the invention, semi-transparent screen 250 is interposed between operator 350 and patient 1. Screen 250 can be constructed with a liquid crystal display or it can be comprised of a partially silvered mirror reflecting an image from a video monitor. Semi-transparent display 250 can be constructed as a relatively large device having dimensions approximately equal to that of the region of interest of patient 1, or alternatively it can be of small dimension and placed relatively close to the operator's eyes, perhaps incorporated into headgear or eyewear.

While several presently preferred embodiments of the novel visualization system have been described in detail herein, many modifications and variations will now become apparent to those skilled in the art. It is, therefore, to be understood that the appended claims are intended to cover all such modifications and variations

## Claims

1. A real-time medical apparatus for displaying to an operator interactive images of internal structures of a patient coinciding with a view by the operator of the patient comprising:
a) a medical imaging system for obtaining multi-dimensional imaging data of internal structures of said patient;
b) a workstation for creating images from the imaging data of internal structures of said patient viewed from a location (x,y,z) and orientation (α,φ,ϑ) which coincide with said view of the patient by said operator;
c) a semi-transparent screen adapted for allowing said operator to view said patient through the semi-transparent screen and adapted for displaying the images of internal structures from the workstation with a desired degree of transparency to create the illusion of internal structures superimposed upon said patient.

2. The real-time medical apparatus of claim 1 further comprising a tracking device adapted to measure the location (x,y,z) and orientation (α,φ,ϑ) of said operator with respect to said patient, for repeatedly providing these measurements to the workstation.

3. The real-time medical apparatus of claim 1 wherein the workstation further comprises surgical planning means adapted to create models of internal structures of said patient, interactively manipulate the models to result in modifications to internal structures, store the models and modifications and display the models and modifications viewed from a location and orientation to coincide with the view of the patient by the operator.

4. The real-time medical apparatus of claim 1 wherein the workstation incorporates normal anatomical models of internal structures, and is adapted to display the models viewed from a location and orientation to coincide with the view of the patient by the operator, such that said workstation can act as a guide in reconstructive surgery.

5. A real-time medical apparatus for displaying to an operator interactive three-dimensional (3D) internal and external images of a patient comprising:
a) a medical imaging system for obtaining three-dimensional (3D) imaging data of internal structures of said patient;
b) tracking means for measuring locations (x₁,y₁,z₁) (x₂,y₂,z₂) and orientation angles (α₁,φ₁,ϑ₁) (α₂,φ₂,ϑ₂) of each eye of said operator with respect to said patient;
c) a first workstation for creating a right computer generated image of internal structures of said patient from the 3D imaging data from the medical imaging system as viewed from the location (x₁,y₁,z₁) and orientation angle (α₁,φ₁,ϑ₁) obtained from the tracking means;
d) a second workstation for creating a left computer generated image of internal structures of said patient from the 3D imaging data from the medical imaging system as viewed from a location (x₂,y₂,z₂) and orientation angle (a₂,φ₂,ϑ₂) obtained from the tracking means;
e) a semi-transparent screen adapted to allow said operator to view said patient through the semi-transparent screen and adapted to display the left and right computer generated images of internal structures from the workstations with a desired degree of transparency in rapid succession;
f) stereoscopic display means synchronized with the semi-transparent screen for allowing only the operator's right eye to view the semi-transparent screen when the right computer generated image is being displayed, and for allowing only the operator's left eye to view the semi-transparent screen when the left computer generated image is being displayed thereby simulating a 3D image of internal and external structures of said patient.

6. A method of aiding an operator to perform a medical procedure upon a patient comprising the steps of:
a) acquiring multi-dimensional medical imaging data from internal structures of said patient;
b) measuring a location (x,y,z) and orientation angle (α,φ,ϑ) of said patient as viewed by said operator;
c) creating a computer generated image of the internal structures from the medical imaging data viewed from the location (x,y,z) and orientation angle (α,φ,ϑ);
d) interposing a semi-transparent screen between said operator and said patient allowing said operator to view said patient through the semi-transparent screen; and
e) displaying the computer generated image on the semi-transparent screen with a desired degree of transparency to create the illusion of internal structures superimposed upon said patient to assist the operator in said medical procedure.

7. The method of aiding an operator in a medical procedure of claim 6 wherein the step of acquiring medical imaging data is performed in real-time by a medical imaging system.

8. The method of aiding an operator in a medical procedure of claim 6 further comprising the step of creating a computer generated guide image of normal anatomical internal structures viewed from a location (x,y,z) and orientation angle (α,φ,ϑ) to be used as a guide to reconstructive surgery; and mixing portions of the guide image with the computer generated images.

9. A method of aiding an operator in a medical procedure of a patient comprising the steps of:
a) obtaining three-dimensional (3D) imaging data of internal structures of said patient;
b) interposing a semi-transparent screen between said operator and said patient allowing said operator to view said patient through the semi-transparent screen;
c) measuring the locations (x₁,y₁,z₁) (x₂,y₂,z₂) and orientation angles (α₁,φ₁,ϑ₁) (α₂,φ₂,ϑ₂) between said patient and each eye of said operator, respectively,
d) creating a left computer generated image and a right computer generated image of internal structures from the 3D imaging data of said patient viewed from distances (x₁,y₁,z₁) (x₂,y₂,z₂) and orientation angles (α₁,φ₁,ϑ₁) (α₂,φ₂,ϑ₂) respectively;
e) displaying the left and right computer generated images of internal structures of a desired degree of transparency in rapid succession on the semi-transparent screen; and
f) blocking the view of the left eye of the operator when the right computer generated image is being displayed, and blocking the view of the right eye of the operator when the left computer generated image is being displayed thereby simulating a 3D image of internal and external structures of said patient.

10. The method of aiding an operator in a medical procedure of claim 9 further comprising the step of adjusting the distances (x₁,y₁,z₁) (x₂,y₂,z₂) and orientation angles (α₁,φ₁,ϑ₁) (α₂,φ₂,ϑ₂) of the left and right computer generated images to correspond to the view of the patient by the operator.
